# EUROPÄISCHE PATENTSCHRIFT

(11) **EP 0 609 274 B1**
(45) Veröffentlichungstag und Bekanntmachung des Hinweises auf die Patenterteilung: **03.09.1997**
(21) Anmeldenummer: 92921122.5
(22) Anmeldetag: 12.10.1992
(51) Int. Cl.: C07H 15/04

(54) **VERFAHREN ZUR HERSTELLUNG HELLFARBIGER ALKYLOLIGOGLYKOSID-PASTEN**
PROCESS FOR PREPARING LIGHT-COLOURED ALKYLOLIGOGLYCOSIDE PASTES
PROCEDE DE FABRICATION DE PATES D'ALKYLOLIGOGLUCOSIDE DE COULEUR CLAIRE

(30) Priorität: 21.10.1991 DE 4134707
(43) Veröffentlichungstag der Anmeldung: 10.08.1994
(73) Patentinhaber: Henkel Kommanditgesellschaft auf Aktien, 40191 Düsseldorf (DE)
(72) Erfinder: WEUTHEN, Manfred, D-5650 Solingen 11 (DE); BECKEDAHL, Burkhard, 40589 Düsseldorf (DE); HARTEL, Irmgard, D-4000 Düsseldorf 13 (DE)
(86) Internationale Anmeldenummer: EP9202343
(87) Internationale Veröffentlichungsnummer: WO9308203

(56) Entgegenhaltungen:
- EP-A- 0 362 671
- EP-A- 0 415 192

## Beschreibung

Die Erfindung betrifft ein Verfahren zur Herstellung hellfarbiger Alkyloligoglykosid-Pasten, bei dem man Glykosen und Fettalkohole bei erhöhter Temperatur in Gegenwart eines sauren Katalysators umsetzt, das Reaktionswasser kontinuierlich entfernt, das Reaktionsprodukt neutralisiert, nichtumgesetzten Fettalkohol destillativ abtrennt und den Rückstand mit Wasser anpastet.

### Stand der Technik

Oberflächenaktive Alkyloliglykoside sind als Rohstoffe für die Herstellung von Waschmitteln bereits seit langem bekannt. Ihre Herstellung erfolgt üblicherweise durch säurekatalysierte Acetalisierung von Zuckern (Glykosen) - insbesondere Glucose - mit Fettalkoholen. Zur Erzielung hoher Ausbeuten wird die Alkoholkomponente in beträchtlichem Überschuß eingesetzt, das freiwerdende Reaktionswasser kontinuierlich aus dem Reaktionsgleichgewicht entfernt und die Reaktion abgebrochen, wenn die Glucose praktisch vollständig, d. h. zu mindestens 99 Gew.-% abreagiert hat. Anschließend wird der saure Katalysator neutralisiert und der überschüssige Fettalkohol destillativ abgetrennt. Stellvertretend für die große Zahl von Verfahren zum Stand der Technik sei hier auf die Druckschriften **US 3,839,318, US 3,450,690, EP 0 132 046 A1** sowie **WO 90/03977** verwiesen.

Ein ernsthaftes technisches Problem bei der Herstellung der Alkyloligoglykoside besteht in der Tatsache, daß die Reaktionsprodukte nach Abtrennung des Fettalkohols eine starke Verfärbung aufweisen, so daß ihre Verwendung als Waschrohstoffe aus ästhetischen Gründen nur nach vorheriger Bleiche in Betracht kommt.

Zur Verbesserung der Farbqualität von oberflächenaktiven Alkyloligoglykosiden schlägt die **EP 0 077 167** vor, daß man bei ihrer Herstellung einen üblichen sauren Katalysator zusammen mit einem sauren Reduktionsmittel aus der Gruppe der phosphorigen Säure, hypophosphorigen Säure, schwefligen Säure, hyposchwefligen Säure, salpetrigen Säure und/oder hyposalpetrigen Säure bzw. der entsprechenden Salze verwendet.

Gemäß der Lehre der **EP 0 102 558** werden hellfarbige C₃-C₅-Alkylglucoside dadurch erhalten, daß man sie in Gegenwart eines sauren Katalysators und mindestens einer dazu äquivalenten Mengen eines Alkalimetallsalzes einer Borsäure, vorzugsweise Natriumperborat, herstellt.

Schließlich wird in der **EP 0 165 721** zur Herstellung farbstabiler Produkte vorgeschlagen, die wäßrige Lösung eines oberflächenaktiven Alkyloligoglucosids zunächst mit einem Oxidationsmittel, vorzugsweise mit einer Wasserstoffperoxidlösung, und anschließend mit einer Schwefeldioxidquelle, z. B. einer wäßrigen Lösung von Natriumbisulfit, zu behandeln.

Aus dem Stand der Technik ist des weiteren bekannt, daß sich nach Abtrennung des Fettalkohols hellfarbige Alkyloligoglykoside erhalten lassen, wenn man die Neutralisation des sauren Katalysators mit Magnesiumoxid durchführt, das in einem Überschuß von 200 bis 500, vorzugsweise 400 Mol.-% - bezogen auf die in der Reaktionsmischung vorhandenen Wasserstoffionen - eingesetzt wird. Hierbei besteht jedoch die Schwierigkeit, daß sich Magnesiumoxid nur schwer in die Reaktionsmischung einarbeiten läßt. Dies ist insbesondere für eine kontinuierliche Arbeitsweise von großem Nachteil, da die Neutralisation mit Magnesiumoxid eine zusätzliche Dispergierstufe erfordert.

Eine weitere Möglichkeit, das Problem der Dispergierung von Magnesiumoxid in der Reaktionsmischung zu umgehen, besteht darin, als Neutralisationsbase wäßrige Natriumhydroxidlösung, gegebenenfalls unter Zusatz von MgO einzusetzen. Die Verwendung von Alkalihydroxiden führt jedoch sowohl bei stöchiometrischer, als auch überstöchiometrischer Arbeitsweise zu verfärbten Produkten; des weiteren treten bei der Destillation starke Schaumprobleme auf, die zusätzliche technische Maßnahmen erforderlich machen und den ganzen Herstellungprozeß aus wirtschaftlicher Sicht belasten. Auch die Bleiche der resultierenden Produkten ist mit herkömmlichen Verfahren nicht oder nur unter großen Schwierigkeiten möglich.

Schließlich besteht ein weiteres Problem, das die wirtschaftliche Herstellung der Alkyloligoglykoside in Frage stellt, in der Tatsache, daß der bei der Destillation anfallende Fettalkohol nicht ohne weiteres in die Acetalisierung zurückgeführt werden kann. Infolge einer Vielzahl von Verunreinigungen ist es vielmehr erforderlich, den Fettalkohol einer reduktiven Behandlung mit Borhydriden zu unterwerfen, da andernfalls die Alkyloligoglykoside sowohl im Hinblick auf ihre Farbqualität, als auch bezüglich ihrer anwendungstechnischen Eigenschaften nachteilig verändert werden.

Die Aufgabe der Erfindung bestand somit darin, ein verbessertes Verfahren zur Herstellung hellfarbiger AlkyloligoglykosidPasten zu entwickeln, das frei von den geschilderten Nachteilen ist.

### Beschreibung der Erfindung

Gegenstand der Erfindung ist ein Verfahren zur Herstellung hellfarbiger Alkyloligoglykosid-Pasten, das sich dadurch auszeichnet, daß man
a) Glykose mit Fettalkoholen in Gegenwart saurer Katalysatoren bei erhöhter Temperatur bis zu einem Restgehalt an Glykose von weniger als 0,1 Gew.-% - bezogen auf die Ausgangsmenge Glykose - umsetzt,
b) freiwerdendes Reaktionswasser kontinuierlich aus dem Gleichgewicht entfernt,
c) die Umsetzungsprodukte mit einer Base neutralisiert, wobei das molare Verhältnis von in der Reaktionsmischung vorhandenen Wasserstoffionen zu zugesetzter Base 1 : 1 bis 1 : 1,5 beträgt,
d) nichtumgesetzten Fettalkohol destillativ abtrennt,
e) den Rückstand anschließend durch Zusatz von Wasser zu einer Paste mit einem Feststoffgehalt von 30 bis 70 Gew.-% - bezogen auf die Paste - verarbeitet, diese alkalisch einstellt und gegebenenfalls in an sich bekannter Weise bleicht.

Überraschenderweise wurde gefunden, daß durch Begrenzung der Menge an nichtumgesetzter Glykose in der Reaktionsmischung auf Werte kleiner 0,1 Gew.-% es möglich wird, die Menge an zur Neutralisation erforderlicher Base signifikant zu vermindern, so daß zuverlässig Alkyloligoglykoside erhalten werden, die schon ohne Bleiche hellfarbig sind.

Die Erfindung beruht auf der Erkenntnis, daß sich Restmengen Glykose im Reaktionsgemisch unter den Destillationsbedingungen u. a. zu Carbonsäuren zersetzen, die ihrerseits Anlaß zur Bildung von unerwünschter Polyglykose und dunkelgefärbter Komponenten geben. Durch eine Verminderung des Restglykosegehaltes im Sinne des erfindungsgemäßen Verfahrens kann die Menge an Base auf das Maß beschränkt werden, das allein zur Neutralisation des sauren Katalysators erforderlich ist; auf einen signifikanten Überschuß an Base zur Neutralisation saurer Abbauprodukte der Glykose kann verzichtet werden, wobei die eingangs geschilderten Probleme entfallen. Das erfindungsgemäße Verfahren erlaubt somit u. a. hellfarbige Alkylglykosid-Pasten mit vermindertem Polyglykosegehalt unter Verwendung geringer Einsatzmengen an Magnesiumoxid auch kontinuierlich herzustellen.

Ein weiterer Vorteil des Verfahrens ist darin zu sehen, daß sowohl der anfallende Fettalkohol, als auch die Alkyloligoglykoside praktisch frei von unerwünschten Abbauprodukten der Glykose und der Alkyloligoglykoside sind. Eine reduktive Nachbehandlung des Fettalkohols ist somit nur noch im Abstand von einigen Reaktionszyklen erforderlich.

Schließlich lassen sich mit den nach dem erfindungsgemäßen Verfahren hergestellten Alkyloligoglykosiden klare oder nur noch schwach getrübte, verdünnte wäßrige Lösungen herstellen, während die Produkte nach dem Stand der Technik häufig austrüben.

Unter **Glykosen**, die als Ausgangsstoffe für die Herstellung von Alkyloligoglykosiden im Sinne des erfindungsgemäßen Verfahrens in Betracht kommen, sind Aldosen bzw. auch Ketosen im weitesten Sinne zu verstehen. Typische Beispiele sind Glucose, Fructose, Mannose, Galactose, Talose, Gulose, Allose, Altrose, Idose, Arabinose, Xylose, Lyxose und Ribose. Vorzugsweise werden wegen der besseren Reaktionsfähigkeit die Aldosen verwendet. Unter den Aldosen kommt wegen ihrer leichten Zugänglichkeit und Verfügbarkeit in technischen Mengen insbesondere die Glucose in Betracht. Die nach dem Verfahren der Erfindung besonders bevorzugt hergestellten Alkyloligoglykoside sind deshalb die Alkyloligoglucoside.

Handelsübliche Glucose enthält in der Regel 1 Mol Kristallwasser. Diese kristallwasserhaltige Glucose kann ohne weiteres verwendet werden. In diesem Fall hat es sich jedoch als zweckmäßig erwiesen, das Kristallwasser zusätzlich, und zwar vor dem Inkontaktbringen mit dem Katalysator aus dem Reaktionsmilieu durch thermische Maßnahmen zu entfernen. Nachdem aber auch wasserfreie Glucose in großen Mengen am Markt erhältlich ist, wird diese bevorzugt in Form eines feinteiligen Pulvers eingesetzt.

Als **saure Katalysatoren** sind generell alle sauren Verbindungen einschließlich der sogenannten Lewis-Säuren geeignet, welche die Acetalisierungsreaktion zwischen Fettalkohol und Zuckermolekül katalysieren. Davon gelten Schwefelsäure, Phosphorsäure, aliphatische und/oder aromatische Sulfonsäuren und sulfosaure Ionenaustauscherharze als besonders geeignet. Für das vorliegende Verfahren werden insbesondere p-Toluolsulfonsäure und/oder Sulfobernsteinsäure, die eine geringe korrodierende Wirkung gegenüber Geräten und Leitungen aus Stahl zeigen, als Katalysatoren bevorzugt. Saure Ionenaustauscherharze sind im vorliegenden Fall ebenfalls geeignet, wenn die Abtrennung des Katalysators nach der Acetalisierung der Glykose vorgesehen ist.

Die sauren Katalysatoren können in Konzentrationen von 0,1 bis 5, vorzugsweise 0,3 bis 2 Gew.-% - bezogen auf den Fettalkohol - eingesetzt werden.

Als **Fettalkohole** kommen primäre Alkohole der Formel **(I)** in Betracht,

**R**^{**1**}**OH (I)**

in der R¹ für einen aliphatischen, linearen oder verzweigten Kohlenwasserstoffrest mit 6 bis 22 Kohlenstoffatomen und 0, 1 2 oder 3 Doppelbindungen steht.

Typische Beispiele hierfür sind Capronalkohol, Caprylalkohol, Caprinalkohol, Laurylalkohol, Myristylalkohol, Cetylalkohol, Palmitoleylalkohol, Stearylalkohol, Oleylalkohol, Elaidylalkohol, Petroselinylalkohol, Linolylalkohol, Linolenylalkohol, Arachylalkohol, Gadoleylalkohol, Behenylalkohol und Erucylalkohol. Bevorzugt sind gesättigte Fettalkohole mit 12 bis 18, insbesondere 12 bis 14 Kohlenstoffatomen.

Wie in der Fettchemie üblich, können die Fettalkohole auch in Form technischer Schnitte vorliegen, wie sie beispielsweise bei der Hochdruckhydrierung von Fettsäuremethylestern auf der Basis von natürlichen Fetten und Ölen anfallen. Als Ausgangsmaterial gleichfalls geeignet sind Oxoalkohole, die über den Weg der ROELEN'schen Oxo-Synthese zugänglich sind. Bevorzugt sind Fettalkoholgemische auf Basis von Kokosöl und Rindertalg.

Die Ansatzbedingungen für die drei Komponenten Glykose, Katalysator und Fettalkohol sind in weiten Grenzen variierbar. So ist es nach einer Variante des erfindungsgemäßen Verfahrens möglich, eine Mischung der Gesamtmengen aller Komponenten vorzulegen und durch Erwärmen die Reaktion einzuleiten. Nach einer anderen Variante wird eine Teilmenge des Fettalkohols mit dem Katalysator vorgelegt und die erwärmte Suspension der Glykose in der restlichen Alkoholmenge nach und nach hinzugefügt. Dabei gibt man im Falle von Laboransätzen einer portionsweisen und im Falle von großtechnischen Ansätzen einer kontinuierlichen Zugabe den Vorzug. Zweckmäßigerweise werden bei der Dosierung die Zeitintervalle zwischen den Dosierportionen so gewählt, daß ständig eine im wesentlichen klare Phase vorliegt, d. h., daß die Menge der nicht umgesetzten Glykose im Reaktionsgemisch gering, d. h. bei nicht mehr als 10 Gew.-%, gehalten wird.

Auch das Ansatzverhältnis Glykose zu Fettalkohol kann in weiten Grenzen variiert werden. Auf diese Weise ist es möglich, den Verteilungsgrad zwischen Alkylmonoglykosid und Alkyloligoglykosiden im Reaktionsprodukt zu steuern. Üblicherweise können Glykose und Fettalkohol im molaren Verhältnis von 1 : 1 bis 1 : 10, vorzugsweise 1 : 2 bis 1 : 6 eingesetzt werden.

Bei Laboransätzen und insbesondere bei den Ansätzen im großtechnischen Maßstab wurde gefunden, daß die Feindispergierung der Glykose im Fettalkohol einen wesentlichen positiven Einfluß auf die Qualität des Reaktionsproduktes hat. Diese Feindispergierung wird dadurch erreicht, daß man die feinpulvrige Glykose, vor allem die Glucose, gegebenenfalls nach einer Feinmahlung, mit dem Fettalkohol intensiv vermischt. Für Laboransätze haben sich dafür die Verwendung eines hochtourigen üblichen Laborrührers oder aber eine Ultraschallbehandlung als geeignet erwiesen. Bei großtechnischen Ansätzen werden zur Feindispergierung vorzugsweise Inline-Mischer, beispielsweise Stator/Rotor-Mischer verwendet. Diese Feindispergierungsmaßnahme hat den erwünschten Nebeneffekt, daß sich die Suspension erwärmt.

Während der Bildung und Abführung des Reaktionswassers wird ein Vakuum von etwa 10 bis 50 mbar angelegt. Während der Reaktion wird die Mischung erwärmt und vorzugsweise ständig durchmischt, was bei Laboransätzen durch einfaches Rühren geschieht, während bei großtechnischen Ansätzen dieses Durchmischen und Erwärmen durch Umpumpen über einen externen Flüssigkeitskreislauf mit einem Wärmeaustauscher erfolgt. Beim Zuführen der Wärmeenergie, die zur Aufrechterhaltung der Reaktionstemperatur benötigt wird, ist es wesentlich, daß zwischen Reaktorwand und Reaktionsgemisch nur eine geringe Temperaturdifferenz vorhanden ist, damit Überhitzungen vermieden werden. Um diese geringe Temperaturdifferenz einzustellen, genügt es bei Ansätzen im Labor, ein übliches Ölbad mit Thermostat zu verwenden und gleichzeitig das Reaktionsgemisch kräftig zu rühren. Bei Ansätzen im technischem Maßstab hat sich als besonders vorteilhaft erwiesen, die Wärmeenergie über einen externen Kreislauf, vorzugsweise bestehend aus einer Pumpe und einem Wärmeaustauscher, vorzunehmen. Zu diesem Zweck wird ständig ein Teil des Reaktionsgemisches über eine Rohrleitung abgezogen, im Wärmeaustauscher erwärmt und wieder in den Reaktor zurückgeführt. Auf diese Weise ist es möglich, hohe Reaktorwandtemperaturen, d. h. solche von mehr als 125 °C zu vermeiden und damit eine negative Auswirkung der Temperaturführung auf die Farbwerte des Endproduktes zu verhindern.

Arbeitet man nach der Dosierungsvariante des Verfahrens, dann legt man vorzugweise 30 bis 70 Gew.-% des Fettalkohols zusammen mit dem Katalysator vor, erwärmt die Mischung auf 80 bis 120 °C und gibt dann die Glykose als Suspension in der erwärmten restlichen Alkoholmenge hinzu, wobei man diese Zugabe vorzugsweise kontinuierlich unter Vakuum durchführt. Das entstehende Reaktionswasser wird ständig abdestilliert. Um die Menge des Reaktionswassers zu bestimmen, kann das Wasser beispielsweise durch Ausfrieren in einer Kühlfalle aufgefangen werden.

In einer weiteren bevorzugten Ausführungsform des Verfahrens, bei der man von der Gesamtmenge des Ansatzes ausgeht, wird so verfahren, daß man zunächst die Mischung aus Fettalkohol und Glykose vorlegt, diese Mischung unter Rühren erwärmt, d. h. bis ca. 80 °C Sumpftemperatur, und dann den sauren Katalysator zu der erwärmten Mischung hinzugibt. Danach wird ein Vakuum angelegt, weiter bis auf ca. 100 bis 120°C erwärmt und das entstehende Reaktionswasser abdestilliert.

Als optimale Temperatur für die Herstellung von Alkyloligoglykosiden hat sich demnach ein Bereich von 80 bis 120 und insbesondere von 100 bis 115°C erwiesen.

Da man, wie bereits ausgeführt, beim erfindungsgemäßen Verfahren die Fettalkohole innerhalb eines weiten Kettenlängenbereiches verwenden kann, läßt sich das Ausmaß des Vakuums auch so einstellen, daß dabei der Siedepunkt des Alkohols um wenigstens 30°C gesenkt wird. Für die Umsetzung der langkettigen Fettalkohole mit 12 bis 18 Kohlenstoffatomen wird das Vakuum vorzugsweise auf einen Wert von 10 bis 50 mbar eingestellt.

Genäß der Lehre des erfindungsgemäßen Verfahrens ist es für die Herstellung hellfarbiger Alkyloligoglykoside essentiell, die Restmenge an Glykose in der Reaktionsmischung zu begrenzen. Ein notwendiges, jedoch nicht hinreichendes Anzeichen für die quantitative Unsetzung der Glykose ist das Ende der Wasserabscheidung. Die Reaktion wird solange fortgesetzt, bis der Rest-Glykosegehalt weniger als 0,1, vorzugsweise weniger als 0,05 Gew.-% beträgt. Die Bestimmung des Glykosegehaltes kann nach an sich bekannten Methoden, beispielsweise diskontinuierlich durch Umsetzung mit FEHLING'scher Lösung oder kontinuierlich mit Hilfe der Fließinjektionsanalyse erfolgen.

Als **Basen** für die Neutralisation des Katalysators kommen Magnesium-, Calcium- und/oder Zinkverbindungen in Betracht. Typische Beispiele sind Calciumhydroxid, Calciumoxid, Magnesiumhydroxid, Magnesiumoxid, die Zeolithe NaA oder NaX, vorzugsweise in Kombination mit Calciumhydroxid, basisches Magnesiumcarbonat, basisches Zinkcarbonat, Calciumcarbonat, Zinkoxid, Magnesiummethylat, Magnesiumethylat, Magnesiumpropylat oder -butylat, d. h. die Alkoholate von niedrigsiedenden Alkoholen, vorzugsweise C₁-C₄-Alkoholen. Besonders bevorzugt ist die Verwendung von Magnesiumoxid.

Die Einsatzmenge der Base richtet sich nach der in der Lösung vorhandenen Konzentration an Wasserstoffionen. Das molare Verhältnis von Wasserstoffionen zu Base kann dabei 1 : 1 bis 1 : 1,5, vorzugsweise 1 : 1,1 bis 1 : 1,3 betragen.

Die **Abtrennung des Fettalkohols** erfolgt mit Hilfe einer Vakuumdestillation. Für die schonende Auftrennung von temperaturempfindlichen Substanzgemischen gilt generell, daß sich Fallfilmverdampfer und insbesondere Dünnschichtverdampfer besonders gut eignen, weil sich in diesen Geräten extrem kurze Verweilzeiten bei den erforderlichen höheren Temperaturen erreichen lassen. Als Dünnschichtverdampfer bezeichnet man solche Verdampfer, in denen ein hochviskoses schwer siedendes Gemisch auf eine beheizte Wand aufgegeben und dort durch rotierende Wischelemente mechanisch verteilt wird. Dabei werden dünne Flüssigkeitsschichten bzw. Flüssigkeitsfilme erzeugt, und die Filmoberflächen ständig erneuert. Die entstehenden Dämpfe strömen entgegen dem Fluß des Produktfilms und verlassen den Verdampfer in den außen angeordneten Kondensator.

Zur Herstellung von Alkyloligoglykosiden mit einem Rest-Fettalkoholgehalt von weniger als 0,1 Gew.-% - bezogen auf das Alkyloligoglykosid - hat es sich als optimal erwiesen, die Destillation mit Hilfe eines Dünnschichtverdampfers bei einer Sumpftemperatur von 120 bis 170°C und einem verminderten Druck von 0,01 bis 1, vorzugsweise 0,05 bis 0,2 mbar durchzuführen.

Die nach dem erfindungsgemäßen Verfahren hergestellten Alkyloligoglykoside stellen Gemische dar, die im wesentlichen aus Alkylmonoglykosid und den Alkyloligoglykosiden, hier im wesentlichen beschränkt auf Di- und Triglykoside, und geringen Anteilen an Tetra- und Pentaglykosiden, bestehen. Die Verteilung zwischen Mono- und Oligoglykosiden in dem Verfahrensprodukt ergibt einen rechnerischen Oligomerisierungsgrad, der zwischen 1 und 5 liegt. Vorzugsweise wird das Verfahren so geführt, daß der Oligomerisierungsgrad zwischen 1 und 1,5 liegt, wobei die Menge an Alkylmonoglykosid, bezogen auf die Gesamtmenge aus Alkylmonoglykosid und Alkyloligoglykosid, deutlich über 50 Gew.-% liegt. (Zur Definition des Oligomerisierungsgrades siehe **Paul J. Flory, Principles of Polymer Chemistry, Cornell University Press, Ithaca, New York, 1953, Seiten 35 bis 37)**. Die Gesamtmenge der übrigen Nebenbestandteile liegt meist unter 10 Gew.-%. Die Restmenge an nicht umgesetzter Glykose beträgt weniger als 0,1 Gew.-% - bezogen auf das Alkyloligoglykosid. Die Anteile an polymerer Glucose im Verfahrensprodukt liegen bei 5 bis 8 Gew.-% -ebenfalls bezogen auf das Alkyloligoglykosid.

Das wasserfreie Alkyloligoglukosid wird mit Wasser zu einer Paste mit einem Feststoffanteil von 40 bis 70 Gew.-% - bezogen auf die Paste - verarbeitet. Sowohl die wasserfreien Alkyloligoglukoside, als auch die daraus erhältlichen Pasten sind hellfarbig. Sofern dies gewünscht wird, können sie nach an sich bekannten Verfahren mit einer Aktivsauerstoffverbindung, insbesondere Wasserstoffperoxid, gebleicht werden. Die Menge der Aktivsauerstoff-Verbindung beträgt dabei im allgemeinen 0,2 bis 1,5 Gew.-%, berechnet als H₂O₂ und bezogen auf die Menge des Produkts nach der Alkoholabtrennung. Da bei dem Bleichvorgang der pH-Wert abnimmt, wird zusammen mit der Perverbindung eine Base, z. B. Natriumhydroxid, zugesetzt, um einen pH-Wert im Bereich von 8 bis 13 aufrechtzuerhalten.

Für eine längere Lagerzeit bzw. einen längeren Transport des pastenförmigen Reaktionsprodukts kann es von Bedeutung sein, daß mikrobielle Abbauprozesse wirksam verhindert werden. Zweckmäßigerweise enthält deshalb das erfindungsgemäß hergestellte pastenförmige Reaktionsprodukt einen die Lagerungsbeständigkeit verbessernden üblichen antimikrobiellen Zusatz in üblicher Menge.

Eine ausreichende Stabilisierung der Paste gegenüber Farbverschlechterung und mikrobiellen Abbau wird schließlich dadurch erzielt, daß man das Produkt durch Zusatz von Alkylihydroxiden auf einen pH-Wert größer 11,5 einstellt.

### Gewerbliche Anwendbarkeit

Die nach dem erfindungsgemäßen Verfahren hergestellten hellfarbigen Alkyloligoglykosid-Pasten zeigen ausgezeichnete Detergenseigenschaften und eignen sich zum Einsatz in Wasch-, Spül- und Reinigungsmittel sowie Produkten zur Haar- und Körperpflege, in denen sie zu 0,1 bis 25, vorzugsweise 1 bis 10 Gew.-% - bezogen auf die Mittel - enthalten sein können.

Die folgenden Beispiele sollen den Gegenstand der Erfindung näher erläutern, ohne ihn darauf einzuschränken.

### Beispiele

### Beispiel 1:

In einem 2-l-Dreihalskolben mit Intensivrührer und Destillationsaufsatz wurden
- 580 g (3 mol): C_{12/14}-Kokosfettalkohol (Lorol^{(R)} Spezial) Fa.Henkel KGaA, Düsseldorf, FRG, Hydroxylzahl 290 und
- 2,2 g (0,01 mol): p-Toluolsulfonsäure
- entsprechend 0,4 Gew.-% bezogen auf den Fettalkohol - vorgelegt. Anschließend wurde die Mischung auf 110 bis 114°C erhitzt und mit einer Suspension von 180 g (1 mol) wasserfreier Glucose (Puridex^{(R)}, Fa.Cerestar Deutschland GmbH) in weiteren 580 g (3 mol) Kokosfettalkohol portionsweise unter Anlegen eines Vakuums von 10 bis 15 mbar versetzt. Das Reaktionswasser wurde über den Destillationsaufsatz aus dem Reaktionsgleichgewicht entfernt und in einer Kühlfalle, die mit flüssigem Stickstoff gekühlt war, ausgefroren und aufgefangen. Es wurden insgesamt 18,2 g Wasser gemessen.

Gegen Ende der Reaktion wurde die Reaktionsmischung weiter bei 110 bis 115°C gerührt, im Abstand von 15 min Proben entnommen und der Glucosegehalt nach der FEHLING'schen Methode bestimmt. Bei einem Restglucosegehalt von weniger als 0,1 Gew.-% wurde die Reaktion abgebrochen.

Danach wurde das abgekühlte Reaktionsgemisch mit 0,3 g (0,007 mol) Magnesiumoxid - entsprechend einem Überschuß an Base von 30 Mol.-% bezogen auf die in der Lösung vorhandenen Wasserstoffionen - versetzt, 30 min gerührt und in eine Dünnschichtdestillationsapparatur überführt. Bei einer Sumpftemperatur von 120 bis 170°C und einem verminderten Druck von 0,1 bar wurden 976 g Kokosfettalkohol ohne Schaumprobleme abgetrennt; der Destillationsrückstand, d.h. das eigentliche Produkt, fiel in einer Menge von 299 g an. Die Kenndaten des Produktes sind in Tab.1 zusammengefaßt. Das wasserfreie Reaktionsprodukt (Hydroxylzahl 656) wurde mit Wasser zu einer Paste mit einem Feststoffgehalt von 50 Gew.-% - bezogen auf die Paste-verarbeitet und mit wäßriger Natriumhydroxidlösung auf pH = 11,7 eingestellt.

### Vergleichsbeispiel V1:

Beispiel 1 wurde wiederholt, die Umsetzung der Glucose mit dem Kokosfettalkohol jedoch bei einem Rest-Glucosegehalt von 0,95 Gew.-% abgebrochen. Das Produkt wurde mit 7,2 g einer 25 gew.-%igen Natriumhydroxidlösung - entsprechend einem Überschuß von 400 Mol.-% bezogen auf die in der Mischung vorhandenen Wasserstoffionen - versetzt, 30 min gerührt und in eine Dünnschichtdestillationsapparatur überführt. Bei einer Sumpftemperatur von 120 bis 170°C und einem verminderten Druck von 0,1 bar wurden 982 g Kokosfettalkohol unter starkem Schäumen abgetrennt; der Destillationsrückstand, d.h. das eigentliche Produkt fiel in einer Menge von 302 g an. Die Kenndaten des Produktes sind in Tab.1 zusammengefaßt. Die Anpastung des Produktes erfolgte analog Bsp.1.

### Vergleichsbeispiel V2:

Vergleichsbeispiel V1 wurde wiederholt, die Neutralisation jedoch mit 2,8 g (0,02 mol) Magnesiumoxid - entsprechend einem Überschuß von 400 Mol.-% bezogen auf die in der Mischungen vorhandenen Wasserstoffionen - durchgeführt. Es wurden 300 g Alkyloligoglyucosid erhalten. Die Kenndaten des Produktes sind in Tab.1 zusammengefaßt. Die Anpastung des Produktes erfolgte analog Bsp.1.

**Tab.1:**

| Zusammensetzung der Reaktionsprodukte Prozentangaben als Gew.-% | | | | | | | | |
|---|---|---|---|---|---|---|---|---|
| Bsp. | Homologenverteilung | | | | FA % | PG % | FZ Klett | Lsg. |
| | MG % | DG % | TG % | HG % | | | | |
| 1 | 68,7 | 15,9 | 5,0 | 3,2 | 0,7 | 6,5 | 70 | klar |
| V1 | 69,7 | 15,5 | 4,9 | 2,1 | 0,8 | 7,0 | 400 | klar |
| V2 | 69,0 | 15,4 | 5,0 | 2,8 | 0,7 | 7,1 | 400 | trübe |
| Legende: MG = Monoglucosid DG = Diglucosid TG = Triglyucosid HG = Höhere Glucoside FA = Fettalkohol PG = Polyglucose FZ = Klett-Farbzahl, 5 gew.-%ige wäßrige Lösung, pH 7, 1-cm-Rundküvette, Blaufilter (400-465 nm) Lsg. = Aussehen einer 10 gew.-%igen wäßrigen Lösung, pH = 10 | | | | | | | | |

## Patentansprüche

1. Verfahren zur Herstellung hellfarbiger Alkyloligoglykosid-Pasten, **dadurch gekennzeichnet,** daß man
a) Glykose mit Fettalkoholen in Gegenwart saurer Katalysatoren bei erhöhter Temperatur bis zu einem Restgehalt an Glykose von weniger als 0,1 Gew.-% - bezogen auf die Ausgangsmenge Glykose - umsetzt,
b) freiwerdendes Reaktionswasser kontinuierlich aus dem Gleichgewicht entfernt,
c) die Umsetzungsprodukte mit einer Base neutralisiert, wobei das molare Verhältnis von in der Reaktionsmischung vorhandenen Wasserstoffionen zu zugesetzter Base 1 : 1 bis 1 : 1,5 beträgt,
d) nichtumgesetzten Fettalkohol destillativ abtrennt,
e) den Rückstand anschließend durch Zusatz von Wasser zu einer Paste mit einem Feststoffgehalt von 30 bis 70 Gew.-% - bezogen auf die Paste - verarbeitet, diese alkalisch einstellt und gegebenenfalls in an sich bekannter Weise bleicht.

2. Verfahren nach Anspruch **dadurch gekennzeichnet,** daß man als Glykose Glucose einsetzt.

3. Verfahren nach den Ansprüchen 1 und 2, **dadurch gekennzeichnet,** daß man Fettalkohole der Formel (I) einsetzt,
**R**^{**1**}**OH (I)**
in der R¹ für einen aliphatischen, linearen oder verzweigten Kohlenwasserstoffrest mit 6 bis 22 Kohlenstoffatomen und 0, 1, 2 oder 3 Doppelbindungen steht.

4. Verfahren nach mindestens einem der Ansprüche 1 bis 3, **dadurch gekennzeichnet,** daß man Glykose und Fettalkohol im molaren Verhältnis von 1 : 1 bis 1 : 10 einsetzt.

5. Verfahren nach mindestens einem der Ansprüche 1 bis 4, **dadurch gekennzeichnet,** daß man als saure Katalysatoren p-Toluolsulfonsäure und/oder Sulfobernsteinsäure einsetzt.

6. Verfahren nach mindestens einem der Ansprüche 1 bis 5, **dadurch gekennzeichnet,** daß man die sauren Katalysatoren in Konzentrationen von 0,1 bis 5 Gew.-% - bezogen auf den Fettalkohol - einsetzt.

7. Verfahren nach mindestens einem der Ansprüche 1 bis 6, **dadurch gekennzeichnet,** daß man die Reaktion bei einer Temperatur von 80 bis 120°C durchführt.

8. Verfahren nach mindestens einem der Ansprüche 1 bis 7, **dadurch gekennzeichnet,** daß man als Basen Magnesium-, Calcium- und/oder Zinkverbindungen einsetzt.

9. Verfahren nach mindestens einem der Ansprüche 1 bis 8, **dadurch gekennzeichnet**, daß man nichtumgesetzten Fettalkohol mit Hilfe der Dünnschichtdestillation bei einer Temperatur von 120 bis 170°C und einem verminderten Druck von 0,01 bis 0,5 bar abtrennt.

## Claims

1. A process for the production of light-colored alkyl oligoglycoside pastes, **characterized in that**
a) glycose is reacted with fatty alcohols in the presence of acidic catalysts at elevated temperature to a residual content of glycose of less than 0.1% by weight, based on the starting quantity of glycose,
b) water of reaction released is continuously removed from the equilibrium,
c) the reaction products are neutralized with a base, the molar ratio of hydrogen ions in the reaction mixture to added base being from 1:1 to 1:1.5,
d) unreacted fatty alcohols is separated by distillation,
e) the residue is subsequently processed by addition of water to a paste having a solids content of 30 to 70% by weight, based on the paste, the paste is alkalized and, optionally, bleached by methods known per se.

2. A process as claimed in claim 1, **characterized in that** glucose is used as the glycose.

3. A process as claimed in claims 1 and 2, **characterized in that** fatty alcohols corresponding to formula **(I)**
**R**^{**1**}**OH (I)**
in which R¹ is an aliphatic, linear or branched hydrocarbon radical containing 6 to 22 carbon atoms and 0, 1, 2 or 3 double bonds,
are used.

4. A process as claimed in at least one of claims 1 to 3, **characterized in that** glycose and fatty alcohol are used in a molar ratio of 1:1 to 1:10.

5. A process as claimed in at least one of claims 1 to 4, **characterized in that** p-toluenesulfonic acid and/or sulfosuccinic acid is/are used as the acidic catalyst(s).

6. A process as claimed in at least one of claims 1 to 5, **characterized in that** the acidic catalysts are used in concentrations of 0.1 to 5% by weight, based on the fatty alcohol.

7. A process as claimed in at least one of claims 1 to 6, **characterized in that** the reaction is carried out at a temperature of 80 to 120°C.

8. A process as claimed in at least one of claims 1 to 7, **characterized in that** magnesium, calcium and/or zinc compounds are used as the bases.

9. A process as claimed in at least one of claims 1 to 8, **characterized in that** unreacted fatty alcohol is separated off by thin-layer distillation at a temperature of 120 to 170°C and under a reduced pressure of 0.01 to 0.5 bar.

## Revendications

1. Procédé pour la fabrication de pâtes d'alkyloligoglycosides de couleur claire,
caractérisé en ce qu'
a) on transforme du glycose avec des alcools gras en présence de catalyseurs acides à une température élevée jusqu'à une teneur résiduelle en glycose inférieure à 0,1 % en poids - par rapport à la quantité initiale de glycose,
b) on élimine l'eau réactionnelle libérée de l'équilibre en continu,
c) on neutralise les produits de transformation avec une base, où le rapport molaire entre les ions présents dans le mélange réactionnel et la base ajoutée est de 1 : 1 jusqu'à 1 : 1,5,
d) on sépare l'alcool gras non transformé par distillation,
e) on transforme le résidu ensuite par addition d'eau en une pâte d'une teneur en matières solides comprise entre 30 et 70 % en poids - par rapport à la pâte -, et on règle celle-ci à un pH alcalin et le cas échéant que l'on décolore de manière elle-même connue.

2. Procédé selon la revendication 1,
caractérisé en ce qu'
on met en oeuvre du glucose comme glycose.

3. Procédé selon les revendications 1 et 2,
caractérisé en ce qu'
on met en oeuvre des alcools gras selon la formule (I),
R¹-OH (I)
dans laquelle R¹ représente un radical aliphatique, linéaire ou ramifié comportant 6 à 22 atomes de carbone et 0, 1, 2 ou 3 doubles liaisons.

4. Procédé selon au moins une revendications 1 à 3,
caractérisé en ce que
le glycose et l'alcool gras sont mis en oeuvre en rapport 1 : 1 jusqu'à 1 : 10.

5. Procédé selon au moins une revendication 1 à 4,
caractérisé en ce que
l'acide p-toluénesulfonique et/ou l'acide sulfosuccinique sont mis en oeuvre comme catalyseurs acides.

6. Procédé selon au moins une revendication 1 à 5,
caractérisé en ce que
les catalyseurs acides sont mis en oeuvre en concentration de 0,1 jusqu'à 5 % en poids - par rapport à l'alcool gras.

7. Procédé selon au moins une revendication 1 à 6,
caractérisé en ce qu'
on réalise la réaction à une température de 80 jusqu'à 120°C.

8. Procédé selon au moins une revendication 1 à 7,
caractérisé en ce qu'
on met en oeuvre des composés de magnésium, de calcium et/ou de zinc comme bases.

9. Procédé selon au moins une revendication 1 à 8,
caractérisé en ce que
l'alcool gras non transformé est séparé à l'aide de distillation à couche mince à une température de 120 jusqu'à 170°C et à une pression réduite de 0,01 bar jusqu'à 0,5 bar.
